# FASCICULE DE BREVET EUROPEEN

(11) **EP 1 942 972 B1**
(45) Date de publication et mention de la délivrance du brevet: **27.09.2017**
(21) Numéro de dépôt: 06808168.6
(22) Date de dépôt: 20.09.2006
(51) Int. Cl.: A61M 25/00, A61B 17/12

(54) **CATHETER DE SECURITE POUR INJECTION DE FLUIDE**
SICHERHEITSKATHETER ZUR INJEKTION VON FLÜSSIGKEITEN
SAFETY CATHETER FOR FLUID INJECTION

(30) Priorité: 05.10.2005 FR 0510154; 20.01.2006 FR 0600502
(43) Date de publication de la demande: 16.07.2008
(73) Titulaire: Balt Extrusion, 95160 Montomorency (FR)
(72) Inventeur: PLOWIECKI, Nicolas, F-95160 MONTMORENCY (FR)
(74) Mandataire: Flavenot, Bernard
(86) Numéro de dépôt international: PCT/FR2006/002146
(87) Numéro de publication internationale: WO 2007/039678

(56) Documents cités:
- US-A1- 2002 082 499
- US-A1- 2002 111 646
- US-A1- 2004 186 464
- US-A1- 2004 225 279
- US-A1- 2005 277 862
- US-B1- 6 296 622
- US-B1- 6 743 251

## Description

La présente invention concerne les cathéters de sécurité pour injection de fluide, notamment embolique, pour des traitements endovasculaires, par exemple pour traiter des malformations artérioveineuses (MAV).

Un tel traitement consiste en l'injection d'un liquide embolique au moyen d'un cathéter, qui permet de boucher le ou les vaisseaux alimentant la zone malade.

Or, il est connu qu'il arrive parfois que ce liquide embolique enveloppe l'extrémité distale du cathéter qui sert à l'injection et l'emprisonne de telle façon qu'il faut tirer sur le cathéter pour tenter de la débloquer.

On conçoit aisément qu'une telle manoeuvre puisse présenter des inconvénients. Par exemple, le liquide embolique est retiré avec le cathéter et ne joue plus son rôle, ou bien il peut être déposé en un endroit non voulu, ce qui peut provoquer la rupture de nombreuses petites artères. Selon un autre inconvénient, l'effort de traction s'avère insuffisant et ne peut être augmenté sans risque de provoquer de grosses lésions artérielles. Dans ce cas, la seule solution est de laisser en place le cathéter presque dans son entier, dans le corps du patient.

Différents systèmes ont été mis au point pour éviter de laisser en place tout le cathéter, par exemple ceux décrits dans les documents suivants : le US 2004/225279 ; le US 2002/165582 et le US-A-6 743 251. Ces systèmes sont essentiellement constitués d'un tube principal terminé à son extrémité distale par un embout abouté au tube principal par un cordon de matière sous forme d'une entretoise qui présente un point de faiblesse pour être facilement détruit par différents moyens. Ils peuvent donner entière satisfaction, à la condition que, lorsque le cathéter est introduit, l'embout ne se détache pas du tube principal avant qu'il ne soit arrivé à l'endroit déterminé pour l'injection du fluide. Le document US 2002/0111646 décrit des systèmes d'introduction d'outils médicaux, par exemple des stents, dans un vaisseau. L'outil médical étant relié au système d'introduction par une connexion qui est dissoute par le sang du patient. Aussi, la présente invention a-t-elle pour but de réaliser un cathéter de sécurité pour injection de fluide, notamment embolique, qui pallie au moins en grande partie les inconvénients possibles mentionnés ci-dessus des cathéters similaires de l'art antérieur.

Plus précisément, la présente invention a pour objet un cathéter de sécurité pour injection de fluide, notamment embolique, comprenant :
- un corps tubulaire défini entre une extrémité proximale et une extrémité distale, ledit corps tubulaire comportant un premier canal débouchant à ses deux première et seconde extrémités respectivement aux deux extrémités proximale et distale du corps tubulaire,
- des moyens d'injection aptes à injecter le fluide dans le premier canal par la première extrémité du dit premier canal,
- un embout tubulaire défini entre deux première et seconde extrémités, ledit embout tubulaire comportant un deuxième canal débouchant par ses deux première et seconde extrémités respectivement aux deux première et seconde extrémités de l'embout tubulaire, et
- des moyens pour réaliser une liaison entre ledit embout tubulaire et ledit corps tubulaire de façon que la seconde extrémité du premier canal et la première extrémité du deuxième canal soient sensiblement coaxiales, ces dits moyens de liaison étant en outre agencés de façon que, en exerçant un couple de forces de valeur déterminée respectivement sur le corps tubulaire et l'embout tubulaire, il y ait rupture de ladite liaison pour obtenir la séparation du corps tubulaire et de l'embout tubulaire,
caractérisé par le fait que les moyens de liaison comportent une bague recouvrant les portions de faces latérales respectivement du corps tubulaire et de l'embout tubulaire au niveau respectivement de l'extrémité distale du corps tubulaire et de la première extrémité de l'embout tubulaire.

D'autres caractéristiques et avantages de la présente invention apparaîtront au cours de la description suivante donnée en regard des dessins annexés à titre illustratif mais nullement limitatif, dans lesquels :
La figure 1 représente le schéma de principe du cathéter de sécurité selon l'invention pour injection de fluide, notamment embolique,
La figure 2 représente une vue en coupe longitudinale partielle d'un mode de réalisation du cathéter de sécurité selon l'invention en accord avec le schéma de principe représenté sur la figure 1, et
La figure 3 représente un perfectionnement du mode de réalisation du cathéter représenté sur la figure 2.

Il est tout d'abord précisé que, sur les figures, les mêmes références désignent les mêmes éléments, quelle que soit la figure sur laquelle elles apparaissent et quelle que soit la forme de représentation de ces éléments. De même, si des éléments ne sont pas spécifiquement référencés sur l'une des figures, leurs références peuvent être aisément retrouvées en se reportant à une autre figure.

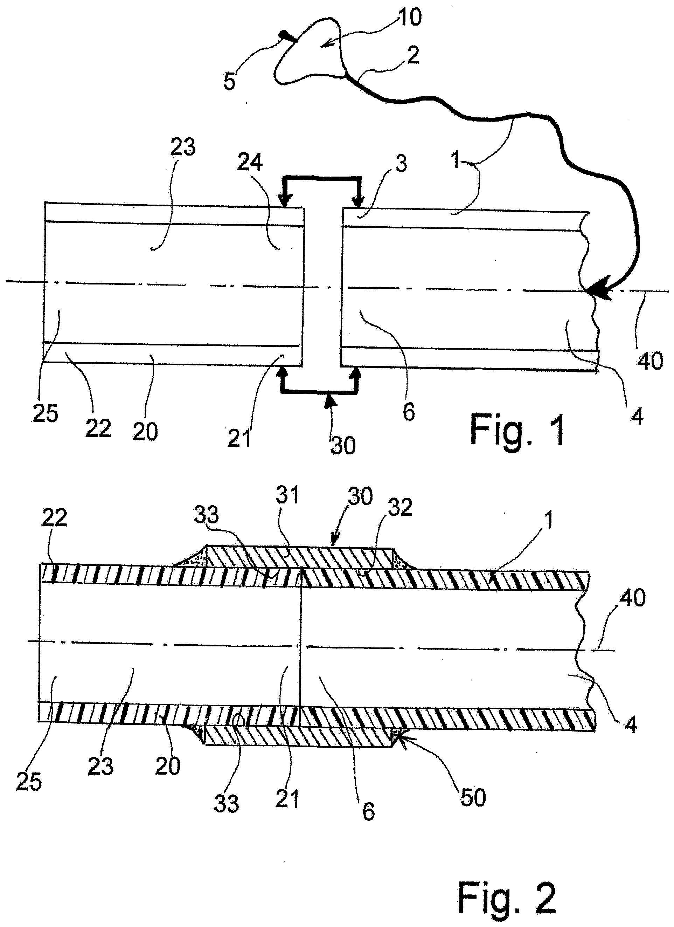

Par référence à la figure 1, le cathéter de sécurité pour injection de fluide, notamment embolique, comprend un corps tubulaire 1 défini entre une extrémité proximale 2 et une extrémité distale 3, le corps tubulaire comportant un premier canal 4 débouchant à ses première et seconde extrémités 5, 6 respectivement aux deux extrémités proximale et distale du corps tubulaire, et des moyens d'injection 10 aptes à injecter le fluide dans le premier canal 4 par sa première extrémité 5.

Ces moyens d'injection 10 sont bien connus en eux-mêmes et ne seront donc pas plus amplement décrits ici, dans l'unique souci de simplifier la présente description.

Le cathéter comporte en outre un embout tubulaire 20 défini entre deux première 21 et seconde 22 extrémités, cet embout tubulaire comportant un deuxième canal 23 débouchant par ses deux première 24 et seconde 25 extrémités respectivement aux deux première et seconde extrémités 21, 22 de cet embout tubulaire 20, et des moyens 30 pour réaliser une liaison de préférence étanche entre le corps tubulaire 1 et l'embout tubulaire 20 de façon que la seconde extrémité 6 du premier canal 4 et la première extrémité 24 du deuxième canal 23 soient sensiblement coaxiales 40 afin que les premier et deuxième canaux 4, 23 forment un canal sensiblement continu, ces moyens de liaison 30 étant en outre agencés de façon que, en exerçant un couple de forces de valeur déterminée respectivement sur le corps tubulaire 1 et l'embout tubulaire 20, il y ait rupture de la liaison, sous toutes formes, pour obtenir la séparation du corps tubulaire 1 et de l'embout tubulaire 20.

Dans une réalisation possible selon l'invention, comme celle plus particulièrement illustrée sur la figure 2 et prise en combinaison avec la représentation selon la figure 1, les moyens de liaison 30 comportent essentiellement une bague 31 en forme de manchon, recouvrant les portions de faces latérales 32, 33 respectivement du corps tubulaire 1 et de l'embout tubulaire 20 au niveau respectivement de l'extrémité distale 3 du corps tubulaire et de la première extrémité 21 de l'embout tubulaire, et avantageusement des moyens d'étanchéité 50 pour réaliser une étanchéité entre la bague 31 et les deux portions de faces latérales 32, 33.

De façon préférentielle, quel que soit le matériau dans lequel sont réalisés le corps tubulaire 1 et l'embout tubulaire 20, la bague 31 est réalisée en platine et les moyens d'étanchéité 50 sont constitués par de la colle liant la paroi latérale de la bague et les deux portions de faces latérales 32, 33 respectivement de l'extrémité distale 3 du corps tubulaire et de la première extrémité 21 de l'embout tubulaire.

Cette colle se présente par exemple, comme illustré sur la figure 2, sous la forme de deux cordons qui réunissent de façon étanche respectivement les deux bords d'extrémité de la bague 31 et les deux portions de faces latérales 32, 33 définies ci-avant.

En général, le matériau dans lequel sont réalisés le corps tubulaire 1 et l'embout 20 est par exemple du polyuréthane, un polyamide, ou un matériau connu sous la Marque "Pebax", ou un mélange de ces trois matériaux.

Sur le schéma selon la figure 1, le corps tubulaire 1 et l'embout tubulaire 20 sont représentés relativement éloignés l'un de l'autre. Mais, de façon avantageuse, comme illustré par exemple sur la figure 2 et dans un but qui sera explicité ci-après, le corps tubulaire et l'embout tubulaire sont aboutés.

La rupture de la liaison peut être obtenue, comme mentionné auparavant, par application d'un couple de forces de valeur déterminée respectivement sur l'embout tubulaire 20 et le corps tubulaire 1. Ce couple de forces est au moins l'un des couples suivants : couple de forces de traction, couple de torsion, combinaison de ces deux types de couple de forces.

Le cathéter selon l'invention décrite ci-dessus, s'utilise de la façon suivante.

Quand il est nécessaire de réaliser une intervention comme celle qui est mentionnée au préambule de la présente description, le cathéter est introduit, par la seconde extrémité 22 de l'embout 20, dans l'artère à soigner, à partir d'un point du corps du patient qui est relativement accessible.

Le corps tubulaire 1 est poussé jusqu'à ce que la seconde extrémité de l'embout arrive à l'endroit à soigner, son extrémité proximale 5 avec les moyens d'injection 10 étant maintenue à l'extérieur du corps du patient.

Il est à souligner que, lors de cette poussée, le corps tubulaire pousse sur l'embout, et qu'il n'y a donc pas de risque de rupture de la liaison entre le corps 1 et l'embout 20, et ce d'autant plus que l'embout est bien maintenu à la fois dans l'axe du corps tubulaire 1 et latéralement, par la bague 31.

Le liquide de traitement est alors injecté dans le cathéter sous une pression déterminée qui, pour permettre l'éjection du liquide par la seconde extrémité 25 du deuxième canal 23, est relativement plus élevée que la pression atmosphérique et/ou la pression sanguine qui règne dans une artère, et se déverse dans l'artère par cette seconde extrémité, à l'endroit dans cette artère choisi par le Praticien.

Si, comme mentionné auparavant, le liquide injecté forme un bouchon autour de la seconde extrémité 22 de l'embout tubulaire 20, bouchon qui empêche le retrait du cathéter, le Praticien exerce une force de traction et/ou une torsion sur l'extrémité proximale du corps tubulaire 1. Sous l'action de cette traction et/ou de cette torsion, parce que les moyens de liaison 30 ont les caractéristiques définies ci-dessus, ou bien l'embout se dégage du bouchon, ou bien il se produit une rupture de la liaison 30 c'est-à-dire une séparation de l'embout tubulaire 20 et du corps tubulaire 1.

Dans le cas de la réalisation selon la figure 2, les moyens de liaison sont généralement agencés de façon que le cordon de soudure et/ou de colle reliant la bague 31 et l'embout 20 se rompe en premier, ce qui permet de séparer l'embout et l'ensemble formé de la bague et du corps tubulaire.

Le cathéter selon l'invention dont un mode de réalisation est illustré sur cette figure 2 présente un avantage important. En effet, la bague 31 constitue une résistance aux déformations latérales des extrémités au contact, du corps tubulaire 1 et de l'embout tubulaire 20, qui pourraient se produire sous la pression du fluide injecté, mais n'exerce pas de résistance à la traction sur le corps tubulaire et ne gêne donc pas la séparation de l'embout 20 et du corps 1 comme expliqué auparavant.

Dans tous les cas, il n'est plus nécessaire de laisser en place la presque totalité du cathéter. Au pire, seul l'embout est laissé en place dans le corps du patient, ne présentant aucun danger.

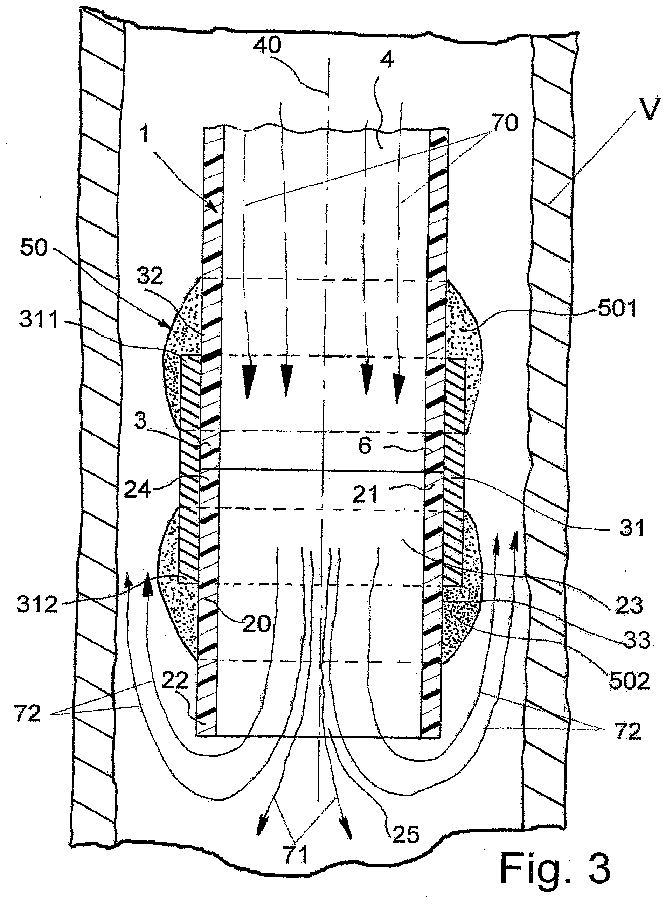

La figure 3 représente un perfectionnement au mode de réalisation du cathéter décrit ci-dessus.

Le cathéter selon ce mode perfectionné comporte les mêmes caractéristiques que celles selon la réalisation décrite en regard de la figure 2, avec en plus le fait qu'au moins une partie de la colle liant la paroi latérale de la bague 31 et les deux portions de parois latérales 32, 33 respectivement de l'extrémité distale 3 du corps tubulaire 1 et de la première extrémité 21 de l'embout tubulaire 20 présente la caractéristique d'être apte à être détruite par le fluide 70, en l'occurrence un liquide de traitement endovasculaire, comme mentionné auparavant.

Dans une réalisation avantageuse, les moyens d'étanchéité définis auparavant sont constitués par deux cordons de colle 501, 502 enrobant au moins partiellement, sinon en totalité, les deux extrémités 311, 312 de la bague 31 et recouvrant respectivement au moins une partie des deux portions de faces latérales 32, 33, de façon préférentielle sur tout leur pourtour, respectivement de l'extrémité distale 3 du corps tubulaire 1 et de la première extrémité 21 de l'embout tubulaire 20.

Selon une caractéristique de l'invention, au moins l'un des deux cordons de colle 501, 502 est apte à être détruit par le fluide 70. De façon préférentielle, le cordon de colle qui est apte à être détruit par le fluide 70 est celui 502 qui est le plus proche de la seconde extrémité 22 de l'embout tubulaire 20, l'autre cordon 501 étant de préférence non destructible par ce fluide 70.

Il est bien précisé que, au sens de la présente description, par "destruction", il est compris l'un des effets suivants : délitement, décomposition, fusion, fracturation, dissolution, que cette "destruction" soit totale ou partielle, de façon qu'elle diminue notablement les propriétés de tenue de la colle pour qu'elle ne puisse plus jouer son rôle d'assemblage solidaire de deux pièces l'une avec l'autre.

Le cathéter selon le mode de réalisation illustré sur la figure 3 et décrit ci-dessus s'utilise et fonctionne de la façon suivante :
Comme décrit précédemment, le corps tubulaire 1 est poussé jusqu'à ce que la seconde extrémité 22 de l'embout tubulaire 20 arrive à l'endroit à soigner, son extrémité proximale 5 avec les moyens d'injection 10 étant maintenue à l'extérieur du corps du patient.

Le fluide 70, c'est-à-dire plus particulièrement le liquide de traitement, est alors injecté et, après avoir parcouru les premier et second canaux 4, 23, s'écoule en 71 par l'extrémité 25 du second canal 23. Cependant, très rapidement, il se produit, devant cette extrémité, un "bouchon" qui entraîne, de facto, un reflux de fluide 72 entre la paroi de la veine ou du vaisseau sanguin V et la paroi extérieure de l'embout tubulaire 20.

Le cordon de colle 502 étant prévu pour être dissout par le fluide 70, il est au moins partiellement éliminé et la bague 31 est apte à être libérée de l'embout tubulaire 20.

Les caractéristiques structurelles de la colle définies ci-dessus permettent, dans un premier temps lors de l'injection du fluide 70, le maintien de l'embout tubulaire 20 et du corps tubulaire 1 aboutés l'un contre l'autre au moyen de la bague 31 collée sur eux et, dans un second temps quand le reflux 72 du fluide se produit, de réduire progressivement la tenue de la colle tout en maintenant la bague 31 encore solidaire de l'embout et du corps tubulaire jusqu'à la fin de l'injection du fluide, et enfin, à la fin de l'injection, le détachement de la bague 31 sous une traction relativement faible exercée sur le corps tubulaire.

Le corps tubulaire 1, la bague 31 et le cordon de colle 501 peuvent alors être retirés, comme explicité auparavant.

La Demanderesse a expérimenté un cathéter selon ce mode de réalisation et a obtenu de très bons résultats avec le liquide embolique connu sous la marque de commerce ONYX^{®} qui comporte un solvant qui permet une diminution d'au moins 50 % de la tenue de la colle lorsque cette dernière est à base de polyuréthane, ce qui est suffisant pour détacher le corps tubulaire 1 de l'embout tubulaire 20 et obtenir le résultat souhaité.

## Revendications

1. Cathéter de sécurité pour injection de fluide, notamment embolique, comprenant :
• un corps tubulaire (1) défini entre une extrémité proximale (2) et une extrémité distale (3), ledit corps tubulaire comportant un premier canal (4) débouchant à ses deux première et seconde extrémités (5, 6) respectivement aux deux extrémités proximale et distale du corps tubulaire,
• des moyens d'injection (10) aptes à injecter le fluide dans le premier canal (4) par la première extrémité (5) du dit premier canal,
• un embout tubulaire (20) défini entre deux première (21) et seconde (22) extrémités, ledit embout tubulaire comportant un deuxième canal (23) débouchant par ses deux première (24) et seconde (25) extrémités respectivement aux deux première et seconde extrémités (21, 22) de l'embout tubulaire (20), et
• des moyens (30) pour réaliser une liaison entre ledit embout tubulaire (20) et ledit corps tubulaire (1) de façon que la seconde extrémité (6) du premier canal (4) et la première extrémité (24) du deuxième canal (23) soient sensiblement coaxiales (40), ces dits moyens de liaison (30) étant en outre agencés de façon que, en exerçant un couple de forces de valeur déterminée respectivement sur le corps tubulaire (1) et l'embout tubulaire (20), il y ait rupture de ladite liaison pour obtenir la séparation du corps tubulaire (1) et de l'embout tubulaire (20),
**caractérisé par le fait que** les moyens de liaison (30) comportent une bague (31) recouvrant les portions de faces latérales (32, 33) respectivement du corps tubulaire (1) et de l'embout tubulaire (20) au niveau respectivement de l'extrémité distale (3) du corps tubulaire (1) et de la première extrémité (21) de l'embout tubulaire (20).

2. Cathéter selon la revendication 1, **caractérisé par le fait qu'**il comporte des moyens d'étanchéité (50) pour réaliser une étanchéité entre ladite bague (31) et les deux portions de faces latérales (32, 33).

3. Cathéter selon la revendication 2, **caractérisé par le fait que** les moyens d'étanchéité (50) sont constitués par de la colle liant la paroi latérale de la bague et au moins une des deux dites portions de faces latérales (32, 33) respectivement de l'extrémité distale (3) du corps tubulaire (1) et de la première extrémité (21) de l'embout tubulaire (20).

4. Cathéter selon la revendication 3, **caractérisé par le fait qu'**au moins une partie de la colle liant la paroi latérale de la bague (31) et au moins une des deux dites portions de faces latérales (32, 33) respectivement de l'extrémité distale (3) du corps tubulaire (1) et de la première extrémité (21) de l'embout tubulaire (20) est apte à être détruite par ledit fluide (70).

5. Cathéter selon la revendication 2, **caractérisé par le fait que** les moyens d'étanchéité sont constitués par deux cordons de colle (501, 502) enrobant au moins partiellement les deux extrémités (311, 312) de ladite bague (31) et recouvrant respectivement au moins une partie des deux dites portions de faces latérales (32, 33) respectivement de l'extrémité distale (3) du corps tubulaire (1) et de la première extrémité (21) de l'embout tubulaire (20).

6. Cathéter selon la revendication 5, **caractérisé par le fait qu'**au moins l'un des deux cordons de colle (501, 502) est apte à être détruit par le fluide (70).

7. Cathéter selon la revendication 6, **caractérisé par le fait que** le cordon de colle apte à être détruit par le fluide (70) est celui (502) qui est le plus proche de la seconde extrémité (22) de l'embout tubulaire (20).

8. Cathéter selon l'une des revendications 5 à 7, **caractérisé par le fait que** ladite colle est apte à être détruite par dissolution.

9. Cathéter selon l'une des revendications 4 et 6, **caractérisé par le fait que** la colle présente des caractéristiques structurelles permettant, dans un premier temps lors de l'injection du fluide (70), le maintien de l'embout tubulaire (20) et du corps tubulaire (1) aboutés l'un contre l'autre au moyen de la bague (31) collée sur eux et, dans un second temps quand un reflux (72) du fluide se produit, de réduire progressivement la tenue de la colle tout en maintenant la bague (31) encore solidaire de l'embout et du corps tubulaire jusqu'à la fin de l'injection du fluide, et enfin, à la fin de l'injection, le détachement de la bague (31) sous une traction relativement faible exercée sur le corps tubulaire.

10. Cathéter selon l'une des revendications 1 à 9, **caractérisé par le fait que** le corps tubulaire (1) et l'embout tubulaire (20) sont aboutés.

11. Cathéter selon l'une des revendications 1 à 10, **caractérisé par le fait que** le couple de forces est au moins l'un des couples suivants : couple de forces de traction, couple de torsion, une combinaison de ces deux couples.

## Patentansprüche

1. Sicherheitskatheter zur Injektion eines Fluids, insbesondere eines embolischen Fluids, umfassend:
• einen rohrförmigen Körper (1), der zwischen einem proximalen Ende (2) und einem distalen Ende (3) definiert ist, wobei der rohrförmige Körper einen ersten Kanal (4) aufweist, der mit seinem ersten und mit seinem zweiten Ende (5, 6) jeweils entsprechend an dem proximalen Ende und dem distalen Ende des rohrförmigen Körpers mündet,
• Injektionsmittel (10), die geeignet sind, das Fluid durch das erste Ende (5) des ersten Kanals in den ersten Kanal (4) einzuspritzen,
• ein rohrförmiges Endstück (20), das zwischen einem ersten Ende (21) und einem zweiten Ende (22) definiert ist, wobei das rohrförmige Endstück einen zweiten Kanal (23) aufweist, der mit seinem ersten Ende (24) und seinem zweiten Ende (25) jeweils entsprechend an dem ersten Ende und dem zweiten Ende (21, 22) des rohrförmigen Endstücks (20) mündet, und
• Mittel (30) zum Herstellen einer Verbindung zwischen dem rohrförmigen Endstück (20) und dem rohrförmigen Körper (1), derart, dass das zweite Ende (6) des ersten Kanals (4) und das erste Ende (24) des zweiten Kanals (23) im Wesentlichen koaxial (40) sind, wobei diese Verbindungsmittel (30) ferner in einer Weise ausgelegt sind, dass bei Ausübung eines Kräftepaars mit bestimmtem Wert jeweils auf den rohrförmigen Körper (1) und das rohrförmige Endstück (20) ein Bruch der Verbindung erfolgt, um die Trennung des rohrförmigen Körpers (1) von dem rohrförmigen Endstück (20) zu erreichen,
**dadurch gekennzeichnet, dass** die Verbindungsmittel (30) einen Ring (31) umfassen, der jeweils die seitlichen Flächenabschnitte (32, 33) des rohrförmigen Körpers (1) und des rohrförmigen Endstücks (20) auf Höhe des distalen Endes (3) des rohrförmigen Körpers (1) und entsprechend des ersten Endes (21) des rohrförmigen Endstücks (20) abdeckt.

2. Katheter nach Anspruch 1, **dadurch gekennzeichnet, dass** er Dichtungsmittel (50) umfasst, um eine Abdichtung zwischen dem Ring (31) und den beiden seitlichen Flächenabschnitten (32, 33) zu erzielen.

3. Katheter nach Anspruch 2, **dadurch gekennzeichnet, dass** die Dichtungsmittel (50) durch den Klebstoff gebildet sind, der die Seitenwand des Rings mit mindestens einem der zwei seitlichen Flächenabschnitte (32, 33) des distalen Endes (3) des rohrförmigen Körpers (1) oder entsprechend des ersten Endes (21) des rohrförmigen Endstücks (20) verbindet.

4. Katheter nach Anspruch 3, **dadurch gekennzeichnet, dass** zumindest ein Teil des Klebstoffs, der die Seitenwand des Rings (31) mit mindestens einem der zwei seitlichen Flächenabschnitte (32, 33) des distalen Endes (3) des rohrförmigen Körpers oder entsprechend des ersten Endes (21) des rohrförmigen Endstücks (2) verbindet, durch das Fluid (70) zerstörbar ist.

5. Katheter nach Anspruch 2, **dadurch gekennzeichnet, dass** die Dichtungsmittel durch zwei Klebstoffstränge (501, 502) gebildet sind, die die beiden Enden (311, 312) des Rings (31) zumindest teilweise umhüllen und jeweils zumindest einen Teil der beiden seitlichen Flächenabschnitte (32, 33) des distalen Endes (3) des rohrförmigen Körpers (1) und entsprechend des ersten Endes (21) des rohrförmigen Endstücks (20) abdecken.

6. Katheter nach Anspruch 5, **dadurch gekennzeichnet, dass** mindestens einer der beiden Klebstoffstränge (501, 502) durch das Fluid (70) zerstörbar ist.

7. Katheter nach Anspruch 6, **dadurch gekennzeichnet, dass** der durch das Fluid (70) zerstörbare Klebstoffstrang jener (502) ist, der am nächsten bei dem zweiten Ende (22) des rohrförmigen Endstücks (20) angeordnet ist.

8. Katheter nach einem der Ansprüche 5 bis 7, **dadurch gekennzeichnet, dass** der Klebstoff durch Auflösen zerstörbar ist.

9. Katheter nach einem der Ansprüche 4 und 6, **dadurch gekennzeichnet, dass** der Klebstoff strukturelle Eigenschaften aufweist, die in einem ersten Zeitabschnitt während der Injektion des Fluids (70) das Halten des rohrförmigen Endstücks (20) und des rohrförmigen Körpers (1) auf aneinander anstoßende Weise mittels des auf sie geklebten Rings (31) ermöglichen und in einem zweiten Zeitabschnitt, in dem ein Rückfluss (72) des Fluids auftritt, das fortschreitende Verringern der Haltewirkung des Klebstoffs unter Aufrechterhaltung der festen Verbindung des Rings (31) mit dem Endstück und dem rohrförmigen Körper bis zum Ende der Injektion des Fluids ermöglichen und schließlich am Ende der Injektion das Lösen des Rings (31) unter einem verhältnismäßig geringen Zug, der auf den rohrförmigen Körper ausgeübt wird, ermöglichen.

10. Katheter nach einem der Ansprüche 1 bis 9, **dadurch gekennzeichnet, dass** der rohrförmige Körper (1) und das rohrförmige Endstück (20) aneinander anstoßen.

11. Katheter nach einem der Ansprüche 1 bis 10, **dadurch gekennzeichnet, dass** das Kräftepaar mindestens eines der folgenden Paare ist: Zugkräftepaar, Torsionskräftepaar, eine Kombination aus diesen beiden Paaren.

## Claims

1. A safety catheter for injecting fluid, in particular embolic fluid, the catheter comprising:
• a tubular body (1) defined between a proximal end (2) and a distal end (3), said tubular body comprising a first channel (4) opening out at its first and second ends (5, 6) respectively at the proximal and distal ends of the tubular body;
• injecter means (10) suitable for injecting the fluid into the first channel (4) via the first end (5) of said first channel;
• a tubular endpiece (20) defined between first and second ends (21, 22), said tubular endpiece having a second channel (23) opening out via its first and second ends (24, 25) respectively at the first and second ends (21, 22) of the tubular endpiece (20); and
• means (30) for making a connection between said tubular endpiece (20) and said tubular body (1) in such a manner that the second end (6) of the first channel (4) and the first end (24) of the second channel (23) are substantially in alignment on an axis (40), said connection means (30) also being arranged in such a manner, that by exerting a force couple of determined value respectively on the tubular body (1) and on the tubular endpiece (20), said connection is broken so as to separate the tubular body (1) and the tubular endpiece (20);
**characterized by** the fact that the connection means (30) comprise a ring (31) covering the side face portions (32, 33) respectively of the tubular body (1) and of the tubular endpiece (20) respectively at the distal end (3) of the tubular body (1) and at the first end (21) of the tubular endpiece (20).

2. A catheter according to claim 1, **characterized by** the fact that it includes sealing means (50) for providing sealing between said ring (31) and the two side face portions (32, 33).

3. A catheter according to claim 2, **characterized by** the fact that the sealing means (50) are constituted by the adhesive bonding the side wall of the ring and at least one of said two side face portions (32, 33) respectively of the distal end (3) of the tubular body (1) and of the first end (21) of the tubular endpiece (20).

4. A catheter according to claim 3, **characterized by** the fact that at least one portion of the adhesive bonding the side wall of the ring (21) with at least one of said two side face portions (32, 33) respectively of the distal end (3) of the tubular body (1) and of the first end (21) of the tubular endpiece (20) is suitable for being destroyed by said fluid (70).

5. A catheter according to claim 2, **characterized by** the fact that the sealing means are constituted by two beads of adhesive (501, 502) covering the two ends (311, 312) of said ring (31) at least in part and covering respectively at least a fraction of said two side face portions (32, 33) respectively of the distal end (3) of the tubular body (1) and of the first end (21) of the tubular endpiece (20).

6. A catheter according to claim 5, **characterized by** the fact that at least one of the two beads of adhesive (501, 502) is suitable for being destroyed by the fluid (70).

7. A catheter according to claim 6, **characterized by** the fact that the bead of adhesive that is suitable for being destroyed by the fluid (70) is the bead (502) that is the closer to the second end (22) of the tubular endpiece (20).

8. A catheter according to any one of claims 5 to 7, **characterized by** the fact that said adhesive is suitable for being destroyed by being dissolved.

9. A catheter according to claim 4 or claim 6, **characterized by** the fact that the adhesive presents structural characteristics making it possible, during injection of the fluid (70), initially to keep the tubular endpiece (20) and the tubular body (1) in abutment one against the other by means of the ring (31) adhesively bonded onto both of them, and subsequently, once a reverse flow (72) of fluid occurs, to reduce progressively the bonding strength of the adhesive, while keeping the ring (31) still secured to the endpiece and the tubular body until the end of fluid injection, and finally, at the end of injection, to detach the ring (31) under relatively weak traction exerted on the tubular body.

10. A catheter according to any one of claims 1 to 9, **characterized by** the fact that the tubular body (1) and the tubular endpiece (20) are in end-to-end abutment.

11. A catheter according to any one of claims 1 to 10, **characterized by** the fact that the force couple is at least one of the following couples: a couple of traction forces; a twisting couple; and a combination of these two couples.
